# EUROPÄISCHE PATENTANMELDUNG

(11) **EP 0 622 362 A1**
(43) Veröffentlichungstag der Anmeldung: **02.11.1994**
(21) Anmeldenummer: 94106198.8
(22) Anmeldetag: 21.04.1994
(51) Int. Cl.: C07D 277/68

(54) **Verfahren zur Herstellung von 2(3H)-Benzothiazolonen**

(30) Priorität: 28.04.1993 DE 4313930
(71) Anmelder: HOECHST AKTIENGESELLSCHAFT, D-65929 Frankfurt am Main (DE)
(72) Erfinder: Forstinger, Klaus, Dr., D-65451 Kelsterbach (DE); Volk, Heinrich, Dr., D-61118 Bad Vilbel (DE); Wykypiel, Werner, Dr., D-63584 Gründau (DE)

(57) **Zusammenfassung**

Verfahren zur Herstellung von gegebenenfalls am Benzolkern substituierten 2(3H)-Benzothiazolonen, indem man ein gegebenenfalls am Benzolkern substituiertes 2-Aminobenzothiazol in Gegenwart von wäßriger Salzsäure gegebenenfalls unter Zusatz eines Chlorids mit einem Diazotierungsmittel zu einem Diazoniumchlorid umsetzt, das Diazoniumchlorid unmittelbar zu einem gegebenenfalls am Benzolkern substituierten 2-Chlorbenzothiazol umsetzt und das 2-Chlorbenzothiazol ohne Zwischenisolierung bei 120°C - 200°C unter Reaktionsdruck hydrolysiert.

## Beschreibung

Die Erfindung betrifft ein Verfahren zur Herstellung von gegebenenfalls substituierten 2(3H)-Benzothiazolonen.

2(3H)-Benzothiazolone stellen wertvolle Zwischenprodukte zur Herstellung von Agrochemikalien wie beispielsweise 4-Chlor-2,3-dihydro-2-oxo-benzothiazol-3-yl-essigsäure dar (British Patent Specification 966,496).

Zur Herstellung von 2(3H)-Benzothiazolonen sind verschiedene Synthesen bekannt:
1) Zyklisierung von 2-Aminothiophenolen mit Phosgen, Chlorkohlensäureestern, Harnstoffen oder Carbonylsulfid;
2) Reduktive Carbonylierung substituierter Nitrobenzole mit Schwefel, Kohlenmonoxid und Wasser in Gegenwart von Basen;
3) Ringschluß von Thiocarbamaten und anschließende Etherspaltung der gebildeten 2-Alkoxybenzthiazole;
4) Oxidation von 2-Mercaptobenzthiazolen oder Benzthiazolylalkylthioethern zu 2-Sulfonyl- bzw. 2-Alkylsulfonylbenzthiazolen und darauffolgender Hydrolyse;
5) Umsetzung von o-Nitrochlorbenzolen mit Thioglykolsäure, Zyklokondensation der gebildeten o-Nitrophenylthioessigsäure mit Acetanhydrid und Entacylierung;
6) Umsetzung von 2-Aminobenzthiazolen mit Alkali- oder Erdalkalihydroxiden in wasserfreiem Medium und Zyklisierung der gebildeten o-Mercaptophenylharnstoffe;
7) JP-021193 beschreibt die Herstellung von 3-substituierten 2(3H)-Benzthiazolonen durch Nitrosierung von 3-substituierten 2(3H)-Iminobenzthiazolen und Hydrolyse in Xylol/Salzsäure.

Die unter 1 - 6 aufgezeigten Verfahren sind mit dem Nachteil behaftet, daß sie auf ökologisch problematischen und/oder zum Teil schwer zugänglichen, damit den Anwendungsbereich einschränkenden, Mercaptoverbindungen basieren.

Das unter 7) aufgeführte Verfahren liefert zwar in sehr guten Ausbeuten 3-substituierte 2(3H)-Benzthiazole, hat aber den Nachteil, daß zum einen toxikologisch bedenkliche N-Nitrosoverbindungen als Zwischenprodukte auftreten und zum anderen in Gegenwart eines Lösemittels (Xylol) gearbeitet wird, was eine kostenintensive, weil autwendige Produktisolation durch Einengen bzw. Abdampfen des Lösemittels und Trocknung lösemittelhaltiger Produkte erforderlich macht. Zudem sind nach diesem Verfahren in 3-Stellung unsubstituierte 2(3H)-Benzothiazolone nicht zugänglich.

Die EP-Anmeldeschrift 245 991 beschreibt die Herstellung von am Stickstoff substituierten Benzothiazolonen. Unter mehreren anderen Möglichkeiten wird hier auch der Weg über 2-Chlor-benzothiazole erwähnt.

In der US-Patentschrift 4 353 919 wird die Hydrolyse von in 2-Stellung Chlor-, Brom- oder Alkoxy-substituierten Benzothiazolen zu Benzothiazolonen beschrieben, wobei allgemein angeführt wird, daß die Hydrolyse in Gegenwart oder Abwesenheit eines inerten, wassermischbaren Lösungsmittels bei Temperaturen von 0 bis 120°C mit einer wäßrigen Mineralsäure durchgeführt wird.

Von den 12 Beispielen beziehen sich lediglich 2 auf die Hydrolyse von 2-Chlorbenzothiazolen. Hierbei wird die Reaktion jeweils mit hochkonzentrierter Salzsäure und Ethanol (1:1 Mischung) durchgeführt, wobei, ausgehend von 2,6-Dichlorbenzothiazol, 6-Chlor-2(3H)-benzothiazolon in 83 %iger Ausbeute und, ausgehend von 2-Chlor-6-fluorbenzothiazol, 6-Fluor-2(3H)-benzothiazolon in 45 %iger Ausbeute erhalten wird.

Ein Beispiel ohne Zusatz eines Lösungsmittels ist nicht aufgeführt.

Es ist literaturbekannt, daß 2-Chlorbenzthiazol in Wasser bis 180°C stabil ist und erst bei Temperaturen ab 200°C unter partieller Zersetzung hydrolysiert (Chem. Ber. 13, 10), während die Umsetzung in Alkohol deutlich einfacher vonstatten geht. Aus Heterocyclic compounds, Volume 5, Seite 550 ist ebenfalls bekannt, daß die direkte Hydrolyse von 2-Chlorbenzthiazolen schwierig ist und langes Erhitzen (25 - 45 Stunden) in alkoholischer Salzsäure erfordert.

Nachteilig an der Verwendung eines Lösungsmittels ist jedoch, daß diese Zusätze organischer Lösungsmittel wie Methanol, Ethanol oder Eisessig zu einer erhöhten Abwasserbelastung mit organischem Material führen.

Es besteht daher ein Bedarf an einem Verfahren, das die vorstehend erwähnten Nachteile vermeidet und sich, ausgehend von den leicht und in großer Vielfalt zugänglichen 2. Aminobenzothiazolen (Herstellung: z.B. EP 116 827), einfach ausüben läßt. Das Verfahren soll technisch ohne großen Aufwand realisierbar sein und aus Gründen der Ökologie möglichst wenig Abfallprodukte liefern. Darüber hinaus soll es die gewünschten Wertprodukte nicht nur in hoher Ausbeute sondern auch in hoher Reinheit zugänglich machen.

Diese Aufgabe wird gelöst durch ein Verfahren zur Herstellung von gegebenenfalls am Benzolkern substituierten 2(3H)-Benzothiazolonen (III). Es ist dadurch gekennzeichnet, daß man ein gegebenenfalls am Benzolkern substituiertes 2-Aminobenzothiazol (I) in Gegenwart von wäßriger Salzsäure gegebenenfalls unter Zusatz eines Chlorids mit einem Diazotierungsmittel zu einem Diazoniumchlorid umsetzt, das Diazoniumchlorid unmittelbar zu einem gegebenenfalls am Benzolkern substituierten 2-Chlorbenzothiazol (II) umsetzt und das 2-Chlorbenzothiazol ohne Zwischenisolierung bei 120 - 200°C unter Reaktionsdruck hydrolysiert. 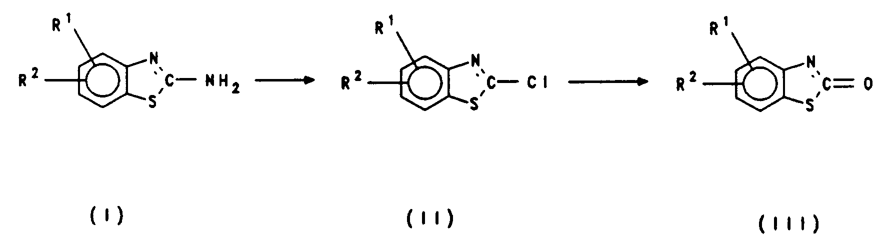
Als Ausgangsmaterial können 2-Aminobenzothiazole der Formel (I) eingesetzt werden, worin R¹ und R² gleich oder verschieden sind und für Wasserstoff, ein Halogen, eine Cyano- oder eine Nitrogruppe stehen.

Bevorzugt ist das Verfahren für 2-Amino-benzothiazole der Formel (I), worin R¹ und R² gleich oder verschieden sind und für Wasserstoff, Fluor, Chlor oder eine Nitrogruppe stehen.

Häufig werden auch 2-Aminobenzothiazole der Formel (I) eingesetzt, worin R¹ Wasserstoff und R² 4-Fluor, 4-Chlor, 4-Nitro, 6-Fluor, 6-Chlor, 6-Nitro, insbesondere 4-Chlor bedeuten.

Die Diazotierung des 2-Aminobenzothiazols wird mit Nitrosylschwefelsäure oder salpetriger Säure, beziehungsweise deren Salze, insbesondere Alkalisalze, bevorzugt Natriumnitrit in einer Konzentration von 100 - 300 Mol.-%, insbesondere 100 - 250 Mol.-%, bezogen auf das eingesetzte 2-Aminobenzothiazol, zusammen mit einer wäßrigen Säure, insbesondere Salzsäure, durchgeführt.

Man arbeitet vorteilhaft in Gegenwart von 15 - 35 %iger, insbesondere 20 - 30 %iger Salzsäure, gegebenenfalls unter Zusatz eines Chlorids bei Temperaturen von 0 bis 50°C.

Als Chloride können Alkali- oder Erdalkalichloride, insbesondere Erdalkalichloride, bevorzugt CaCl₂, eingesetzt werden.

Nach beendeter Reaktion wird üblicherweise das überschüssige Nitrit (Diazotierungsmittel) durch Zusatz von Harnstoff oder Amidosulfonsäure, bevorzugt als wäßrige Lösungen oder anderen zur Zerstörung von überschüssigem Nitrit (Diazotierungsmittel) geeigneten Reagentien entfernt.

Das resultierende Reaktionsgemisch wird anschließend ohne weiteren Zusatz eines Lösungsmittels, insbesondere eines organischen Lösungsmittels im geschlossenen Druckgefäß bei Temperaturen von 120 bis 200°C hydrolysiert. In vielen Fällen hat es sich bewährt, bei Temperaturen von 130 bis 180°C, insbesondere 130 bis 150°C, bevorzugt 135 bis 150°C zu arbeiten. Die erforderliche Reaktionszeit hängt einerseits von der Größe des Ansatzes und andererseits auch von jeweiligen Temperaturen ab. Im allgemeinen hat sich eine Reaktionszeit von 1 bis 10, insbesondere 3 bis 5 Stunden als ausreichend erwiesen. Die Reaktion wird unter Druck, insbesondere unter dem sich jeweils einstellenden Reaktionsdruck durchgeführt. Üblicherweise beträgt der Druck 1,5 bis 20, insbesondere 2 bis 16 bar.

Nach Abkühlen auf 20°C wird das feinkristalline Produkt auf übliche Weise isoliert und getrocknet.
Man erhält auf diese Weise 2(3H)-Benzthiazolone in 82 - 94 %iger Ausbeute, was einer Ausbeute je Reaktionsstufe von 90 bis 97 % entspricht.

Die so erzielten Ausbeuten sind über 2 Stufen (ausgehend von 2-Aminobenzothiazolen) deutlich höher als in US-PS 4 353 919 für die 1-Stufen Reaktion (ausgehehend von den 2-Chlor-benzothiazolen) beschrieben.
6-Fluor-2(3H)-benzothiazolon wird nach dem erfindungsgemäßen Verfahren in einer Ausbeute von 85 % (gegenüber 45 % in US-PS 4 353 919), 6-Chlor-2(3H)-benzothiazolon in einer Ausbeute von 91,6 % (gegenüber 83 % in US-PS 4 353 919) erhalten.

Das erfindungsgemäße Verfahren ist insofern für eine technische Anwendung besonders geeignet, da es einerseits auf eine ausbeutemindernde und kostenintensive Zwischenisolierung von 2-Chlorbenzthiazolen verzichtet und andererseits nur relativ kurze Reaktionszeiten erfordert. Zudem stellt es ein umweltfreundliches Verfahren dar, da es vollständig ohne abwasserbelastende Lösungsmittel auskommt und außerdem 2(3H)-Benzthiazolone in sehr hoher Ausbeute und guter Qualität liefert.

Falls gewünscht, kann das Verfahren auch auf die isolierten 2-Chlor-benzothiazole der Formel (II) angewandt werden, indem diese in Mineralsäuren ohne Zusatz von Lösungsmitteln unter Reaktionsdruck bei 120 bis 200°C, insbesondere 130 bis 180°C, bevorzugt 130 bis 150°C hydrolysiert werden.

Hierfür können Chlorbenzothiazole der Formel (II) eingesetzt werden, worin R¹ und R² gleich oder verschieden sind und für Wasserstoff, Halogen, Cyano oder Nitrogruppe stehen.

Die nachstehenden Beispiele dienen zur Erläuterung des erfindungsgemäßen Verfahrens ohne es darauf zu beschränken.

### Beispiel 1

In einem 2 l-Druckgefäß werden 768 g Salzsäure 30 %ig vorgelegt und bei Raumtemperatur 195,4 g 2-Amino-4-chlorbenzthiazol feucht mit einem Trockengehalt von 50,92 % eingetragen. Nach Zusatz von 71,3 g Calciumchlorid wird 30 min. auf 50°C erhitzt und wieder auf 20°C abgekühlt.
Anschließend werden bei 20 - 24°C innerhalb 8 Stunden 174,4 g wäßrige 40 %ige Natriumnitritlösung gleichmäßig bei guter Rührung unter die Oberfläche dosiert. Nach Dosierende wird 2 Stunden bei 20 - 24°C nachgerührt. Danach wird überschüssiges Nitrit durch langsamen Zusatz von ca. 40 g wäßriger 18 %iger Harnstofflösung zerstört. Anschließend wird das Reaktionsgefäß geschlossen und unter gutem Rühren das Reaktionsgemisch 3 bis 5 Stunden auf 130 - 135°C (Druck 4 - 5 bar) erhitzt. Nach Abkühlen auf 20°C wird das feinkristalline Produkt abgesaugt, neutral gewaschen und bei 60°C bis zur Gewichtskonstanz getrocknet.

Man erhält 96,8 g 4-Chlorbenzthiazol-2-on mit einem Reingehalt von 97 % (entspr. 93,9 % Ausbeute). Alkalisch ungelöstes Produkt besitzt einen DSC-Schmelzpunkt von 203-203,5°C.

### Beispiel 2

In einem 1000 ml-Druckgefäß werden 384 g Salzsäure 30 %ig vorgelegt und bei Raumtemperatur 50,3 g 2-Amino-6-chlorbenzthiazol (Reingehalt: 99,2 %) eingetragen. Nach Zusatz von 35,7 g Calciumchlorid wird 30 min. auf 50°C erhitzt und wieder auf 20°C abgekühlt. Anschließend werden bei 20 - 24°C innerhalb 5 Stunden 87,2 g wäßrige 40 %ige Natriumnitritlösung gleichmäßig bei guter Rührung unter die Oberfläche dosiert. Nach Dosierende wird 1 Stunde bei 20 - 24°C nachgerührt. Danach wird überschüssiges Nitrit durch langsamen Zusatz von ca. 20 g wäßriger 18 %iger Harnstofflösung zerstört. Anschließend wird das Reaktionsgefäß geschlossen und unter gutem Rühren das Reaktionsgemisch 4 Stunden auf 145 - 150°C (Druck 5 - 6 bar) erhitzt. Nach Abkühlen auf 20°C wird das beige Produkt abgesaugt, neutral gewaschen und bei 60°C bis zur Gewichtskonstanz getrocknet. Man erhält 46,4 g 6-Chlorbenzthiazol-2-on mit einem Reingehalt von 99 % (entspr. 91,6 % Ausbeute). Alkalisch umgefälltes Produkt besitzt einen DSC-Schmelzpunkt von 211,2°C.

### Beispiel 3

In einem 750 ml Druckgefäß werden bei Raumtemperatur 28,75 g 2-Amino-6-nitrobenzthiazol, 210 g Salzsäure 30 %ig und 29,5 g Wasser vorgelegt und anschließend auf 50°C erhitzt. Nach 30 Minuten Rühren wird das Reaktionsgemisch auf 20°C abgekühlt. Anschließend werden bei 20 - 30°C unter sehr gutem Rühren 50,75 g wäßrige Natriumnitritlösung innerhalb 3 Stunden gleichmäßig zudosiert. Nach Dosierende wird 2,5 Stunden bei 20 - 30°C nachgerührt. Danach wird überschüssiges Nitrit durch Zugabe von ca. 15 g 18 %iger wäßriger Harnstofflösung entfernt. Im Anschluß daran wird das Reaktiongefäß geschlossen und das Reaktionsgemisch 5 Stunden auf 140 - 150°C erhitzt (Druck ca. 4 bar). Nach dem Abkühlen auf 20°C wird das kristalline Produkt abgesaugt, neutral gewaschen und bei 60°C bis zur Gewichtskonstanz getrocknet. Man erhält 27,3 g 6-Nitrobenzthiazol-2-on mit einem Reingehalt von 97,8 % (entspr. 92,4 % Ausbeute). Alkalisch umgelöstes Produkt besitzt einen DSC-Schmelzpunkt von 252,5°C.

### Beispiel 4

In einem 750 ml Druckgefäß werden bei Raumtemperatur 33 g 2-Amino-4,5-dichlorbenzthiazol, 241 g Salzsäure 30 %ig und 33,9 g Wasser vorgelegt und anschließend auf 50°C erhitzt. Nach 30 Minuten Rühren werden bei 50°C unter gutem Rühren 58,3 g wäßrige, 40 %ige Natriumnitritlösung gleichmäßig in 3 Stunden zudosiert. Nach Dosierende wird 4 Stunden bei 50°C nachgerührt. Danach wird überschüssiges Nitrit durch Zugabe von ca. 15 g 18 %iger wäßriger Harnstofflösung entfernt. Im Anschluß daran wird das Reaktionsgefäß geschlossen und das Reaktionsgemisch 4 Stunden auf 140 - 150°C erhitzt (Druck ca. 6 bar). Nach dem Abkühlen auf 20°C wird das kristalline Produkt abgesaugt, mit Wasser gewaschen und bei 60°C bis zur Gewichtskonstanz getrocknet. Man erhält 27,9 g 4,5-Dichlorbenzthiazol-2-on mit einem Reingehalt von 97,2 % (entspr. 81,8 % Ausbeute) mit einem DSC-Schmelzpunkt von 229,1°C.

### Beispiel 5

In einem 750 ml Druckgefäß werden bei Raumtemperatur 22,4 g 2-Amino-6-fluorbenzthiazol (Reingehalt: 97 %) und 191,1 g Salzsäure 30 %ig vorgelegt. Anschließend wird für 30 Minuten auf 50°C erhitzt und danach auf 10°C abgekühlt. Danach werden bei 10°C unter gutem Rühren 46,7 g wäßrige 40 %ige Natriumnitritlösung gleichmäßig innerhalb 1 Stunde zudosiert. Nach Dosierende wird das Reaktionsgemisch 12 Stunden bei 20°C nachgerührt. Danach wird überschüssiges Nitrit durch Zugabe von ca. 12,5 g 18 %iger wäßriger Harnstofflösung entfernt. Im Anschluß daran wird das Reaktionsgefäß geschlossen und das Reaktionsgemisch 5 Stunden auf 140 - 150°C erhitzt (Druck ca. 4 - 5 bar).

Nach dem Abkühlen auf 20°C wird das graue, kristalline Produkt abgesaugt, mit wenig Wasser gewaschen und bei 60°C bis zur Gewichtskonstanz getrocknet. Man erhält 18,9 g 6-Fluorbenzthiazol-2-on mit einem Reingehalt von 97,7 % (entspr. 85 % Ausbeute). Alkalisch umgelöstes Produkt besitzt einen Schmelzpunkt von 188,4°C.

## Patentansprüche

1. Verfahren zur Herstellung von gegebenenfalls am Benzolkern substituierten 2(3H)-Benzothiazolonen, dadurch gekennzeichnet, daß man ein gegebenenfalls am Benzolkern substituiertes 2-Aminobenzothiazol in Gegenwart von wäßriger Salzsäure gegebenenfalls unter Zusatz eines Chlorids mit einem Diazotierungsmittel zu einem Diazoniumchlorid umsetzt, das Diazoniumchlorid unmittelbar zu einem gegebenenfalls am Benzolkern substituierten 2-Chlorbenzothiazol umsetzt und das 2-Chlorbenzothiazol ohne Zwischenisolierung bei 120°C - 200°C unter Reaktionsdruck hydrolysiert.

2. Verfahren nach Anspruch 1, dadurch gekennzeichnet, daß man ein 2-Aminobenzothiazol der Formel I 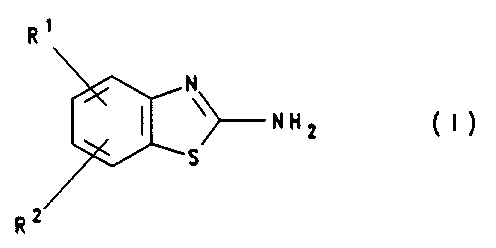 worin R¹ und R² gleich oder verschieden sind und für Wasserstoff, Halogen, Cyano- oder Nitrogruppe stehen, einsetzt.

3. Verfahren nach Anspruch 1, dadurch gekennzeichnet, daß man ein 2-Aminobenzothiazol der Formel (I), worin R¹ und R² gleich oder verschieden sind und für Wasserstoff, Fluor, Chlor oder eine Nitrogruppe stehen, einsetzt.

4. Verfahren nach Anspruch 1, dadurch gekennzeichnet, daß man ein 2-Aminobenzothiazol der Formel I, worin R¹ Wasserstoff und R² Fluor, Chlor oder eine Nitrogruppe bedeuten, einsetzt.

5. Verfahren nach Anspruch 1, dadurch gekennzeichnet, daß man ein 2-Aminobenzothiazol der Formel I, worin R¹ Wasserstoff und R² 4-Fluor, 4-Chlor, 4-NO₂ bedeuten, einsetzt.

6. Verfahren nach Anspruch 1, dadurch gekennzeichnet, daß man ein 2-Aminobenzothiazol der Formel I, worin R¹ Wasserstoff und R² 6-Fluor, 6-Chlor, 6-NO₂ bedeuten, einsetzt.

7. Verfahren nach Anspruch 1, dadurch gekennzeichnet, daß man 2-Amino-4-chlorbenzthiazol einsetzt.

8. Verfahren nach einem oder mehreren der Ansprüche 1 bis 7, dadurch gekennzeichnet, daß man die Salzsäure in einer Konzentration von 15 bis 35, insbesondere 20 bis 30 Gew.-%, einsetzt.

9. Verfahren nach einem oder mehreren der Ansprüche 1 bis 8, dadurch gekennzeichnet, daß man als Chlorid ein Alkali- oder Erdalkalichlorid, insbesondere Erdalkalichlorid, bevorzugt CaCl₂ einsetzt.

10. Verfahren nach einem oder mehreren der Ansprüche 1 bis 9, dadurch gekennzeichnet, daß man als Diazotierungsmittel Nitrosylschwefelsäure oder salpetrige Säure und deren Salze, insbesondere Alkalisalze, bevorzugt Natriumnitrit, einsetzt.

11. Verfahren nach einem oder mehreren der Ansprüche 1 bis 10, dadurch gekennzeichnet, daß man ohne weiteren Zusatz eines Lösungsmittels, insbesondere eines organischen Lösungsmittels, hydrolysiert.

12. Verfahren nach einem oder mehreren der Ansprüche 1 bis 11, dadurch gekennzeichnet, daß man bei Temperaturen von 130 - 180°C, insbesondere 130 - 150°C, bevorzugt 135 - 150°C, hydrolysiert.

13. Verfahren zur Herstellung von gegebenenfalls am Benzolkern substituierten 2-(3H)-Benzothiazolonen, dadurch gekennzeichnet, daß man ein gegebenenfalls am Benzolkern substituiertes 2-Chlorbenzothiazol in Mineralsäuren, insbesondere Salzsäure, Schwefelsäure oder Phosphorsäure ohne Zusatz von Lösungsmitteln, insbesondere organischen Lösungsmitteln, unter Reaktionsdruck bei 120 - 200°C, insbesondere 130 - 180°C, bevorzugt 135 - 150°C, hydrolysiert.

14. Verfahren nach Anspruch 13, dadurch gekennzeichnet, daß man ein 2-Chlorbenzothiazol der Formel II 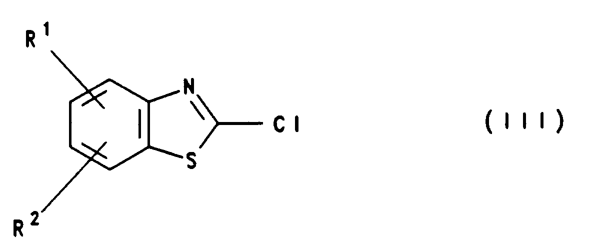 worin R¹ und R² gleich oder verschieden sind und für Wasserstoff, Halogen, Cyano oder Nitrogruppe stehen, einsetzt.
